# EUROPÄISCHE PATENTSCHRIFT

(11) **EP 2 094 213 B1**
(45) Veröffentlichungstag und Bekanntmachung des Hinweises auf die Patenterteilung: **02.09.2015**
(21) Anmeldenummer: 07819712.6
(22) Anmeldetag: 09.11.2007
(51) Int. Cl.: A61F 13/02, A61K 9/70, A61K 47/32, A61L 15/16

(54) **TRANSDERMALES THERAPEUTISCHES SYSTEM MIT HOHER WIRKSTOFFAUSNUTZUNGSRATE UND DOSIERGENAUIGKEIT**
TRANSDERMAL THERAPEUTIC SYSTEM WITH HIGH RATE OF UTILIZATION OF ACTIVE SUBSTANCE AND DOSING ACCURACY
SYSTÈME THÉRAPEUTIQUE TRANSDERMIQUE AVEC UN TAUX D'UTILISATION DE LA SUBSTANCE ACTIVE ÉLEVÉ ET UNE POSOLOGIE PLUS PRÉCISE

(30) Priorität: 21.11.2006 DE 102006054733
(43) Veröffentlichungstag der Anmeldung: 02.09.2009
(73) Patentinhaber: LTS LOHMANN Therapie-Systeme AG, 56626 Andernach (DE)
(72) Erfinder: HORSTMANN, Michael, 56564 Neuwied (DE); MÜLLER, Walter, 56626 Andernach (DE)
(74) Vertreter: Schweitzer, Klaus
(86) Internationale Anmeldenummer: PCT/EP2007/009707
(87) Internationale Veröffentlichungsnummer: WO 2008/061639

(56) Entgegenhaltungen:
- WO-A-02/41878
- WO-A-02/069940
- DE-A1- 10 012 908
- DE-A1- 10 103 860
- DE-A1-102006 026 060

## Beschreibung

Transdermale Therapeutische Systeme (TTS) sind seit einer Reihe von Jahren in die Therapie eingeführt. Zur Referenz für die Anwendung insbesondere im Bereich der Schmerztherapie wird auf die Literatur (B. Asmussen, Transdermale Therapeutische System-Eigenschaften und Anwendungen; In: Likar, Rudolf: Praxis der transdermalen Schmerztherapie, 1. Auflage-Bremen 2002) verwiesen.

Insbesondere für hochaktive Wirkstoffe mit Tagesdosen von unter 30 mg, sogar noch unter 5 mg, sind in den letzten Jahren eine Reihe von Wirkstoffen in die transdermale Therapie eingeführt worden: Nikotin, Fentanyl, Buprenorphin, Nitroglycerin, Estradiol, Rotigotine, um nur einige Beispiele zu nennen. Für all diese Stoffe sind flache Systeme unter Verwendung mindestens einer der Polymergruppen Silikonpolymere, Polyisobutylen-Klebemassen oder Polyacrylat-Klebemassen verwendet worden. Wegen der günstigen Möglichkeiten, durch entsprechende Derivatisierung auf Wirkstofferfordernisse zu reagieren und wegen der allgemein ohne weitere Zusatzstoffe ausreichenden Verklebung auf die Haut werden insbesondere Acrylatpolmere bevorzugt für transdermale therapeutische Systeme verwendet. Beispiele hierfür sind insbesondere das neu eingeführte Durogesic SMAT®, sowie zahlreiche generische Fentanyl-TTS, welche von den Firmen Ratiopharm und Hexal in Deutschland in den Jahren 2005 und 2006 in den Markt eingeführt wurden. Aber auch andere Wirkstoffe sind bevorzugt mit dem polymeren Prinzip AcrylsäureCopolymer auf der menschlichen Haut verfügbar gemacht worden. So sind transdermale therapeutische Systeme mit Nikotin (Nicotinel®-TTS), Buprenorphin (Transtec®; Grünenthal) und Estradiol (Estraderm MX®, Novartis) in den Markterfolgreich eingeführt worden.

Systemaufbauten solcher transdermalen Systeme enthalten eine oder mehrere Schichten, wobei in früheren Jahren insbesondere dem Aspekt der Zufuhrkontrolle durch die Systeme selbst besondere Rechnung getragen wurde. Typische Aufbauten früherer Zeiten haben daher eine Trennung in Klebschicht, Membranschicht und Reservoirschicht vorgesehen, bei denen der wesentliche Wirkstoffanteil in der Reservoirschicht enthalten war.

All diesen Systemen ist gemeinsam, daß häufig nur ein sehr kleiner Teil des zur Verfügung stehenden Wirkstoffes transdermal verabreicht werden kann, die sogenannte Systemausnutzungsrate also relativ gering ist. Dies hat insbesondere bei transdermalen therapeutischen Systemen Bedeutung, welche teure Wirkstoffe enthalten, insbesondere neue synthetische Wirkstoffe wie Rotigotine, Fentanyl, Buprenorphin oder Sufentanil.

Dem Anspruch, eine hohe Wirkstoffausnutzungsrate zu erreichen, steht die Erfordernis einer für die Klebkraft und Herstellbarkeit erforderlichen großen Schichtdicke (Auftragsgewicht) der Klebeschichten und Reservoirschichten entgegen, sowie die Eigenschaft der meisten Klebmassen, einen hohen Wirkstoffanteil zu lösen, bevor die für den transdermalen Einsatz notwendige hohe thermodynamische Aktivität (nahe der Sättigungslöslichkeit) erreicht wird.

Das Erfordernis großer Schichtdicken zusammen mit hohen Sättigungslöslichkeiten der Wirkstoffe in den Polymeren bedingt den leider unerwünscht hohen Wirkstoffeinsatz der meisten transdermalen therapeutischen Systeme. Beispielhaft seien für den Stand der Technik im Bereich transdermaler Systeme mit beschränkter Wirkstoffausnutzung z.B. genannt US 5, 240,711, bei welchem unter Zugabe weiterer Bestandteile Polyacrylatmatrices mit 0,1-20% Buprenorphin-Base beschrieben sind. US 6,090,405 beschreibt transdermale Systeme für Buprenorphin, welche ein Acrylat-Copolymer und darin enthaltene quervernetzte Acrylpolymerpartikel enthalten. WO 03/018075 beschreibt ein transdermales therapeutisches System mit Fentanyl oder verwandten Substanzen als Wirkstoff, welches eine Matrixschicht auf Basis von Polyacrylat enthält. Aus Gründen der höheren Wirkstoffausnutzungsrate wird hier ein acrylsäuregruppenfreies Copolymer ausgewählt, da die durch Ionenpaareffekte geförderte Löslichkeit von Fentanyl und ähnlichen Wirkstoffen durch Carboxylgruppen sonst allzu stark erhöht wird.

WO 03/097020 beschreibt ein Zweischicht-System, bei welchem die hautseitige Schicht eine geringere Wirkstoffaffinität aufweist als die hautferne Schicht und gleichzeitig eine größere Schichtdicke aufweist.

Zielsetzung gemäß der technischen Lehre dieser Publikation ist das Erreichen einer konstanten Abgaberate über längere Zeitperioden. Eine geringe Ausnutzungsrate durch verbleibenden Wirkstoff in der besser lösenden hautfernen Schicht wird damit durchaus weiterhin hingenommen.

EP 1 137 406 B1 beschreibt ein Pflaster zur transdermalen Applikation von flüchtigen Wirkstoffen, insbesondere von Nikotin. Das Pflaster ist zweischichtig, wobei die hautferne Schicht aus einem wirkstoffhaltigen Silikonklebermaterial, die Hautkontaktschicht aus einem Acrylkleber besteht und beide Schichten ungefähr die gleiche Dicke aufweisen. Es wird darauf hingewiesen, daß nach dem Laminieren und Erreichen eines Gleichgewichts die Acrylkleberschicht ebenfalls einen Teil des Wirkstoffs und zwar ungefähr 2,5 bis 3,0 Gew.-% enthält.

Neben den vorbeschriebenen wirtschaftlichen Faktoren spielen auch zunehmend restriktive Anforderungen der Zulassungsbehörden an die Dosiergenauigkeit von transdermalen therapeutischen Systemen eine große Rolle. So lassen sich bei wirtschaftlich durchführbaren Dosierverfahren Beschichtungs-Genauigkeiten mit einer Standardabweichung von etwa +/- 5 g/m² durchaus erreichen. Bei nach dem Stand der Technik üblichen Flächengewichten von etwa 50-100 g/m² bleiben demnach die Streuungen für die Beschichtungsgenauigkeit und später den Wirkstoffgehalt der gestanzten TTS im Bereich zwischen 5 und 10%. Diese Genauigkeit reichte bei bisherigen pharmazeutischen Zulassungsverfahren durchaus aus. Mit der im Jahre 2005 revidierten, Monographie des Europäischen Arzneibuchs werden jedoch Genauigkeiten mit bis zu einer Standardabweichung von unter 3% gefordert, wenn die Mittenlage des erreichten Schichtgewichtes bis 10% vom geforderten Wert abweicht. Diese Erfordernis macht es somit nicht mehr ökonomisch möglich, mit Schichtdicken von etwa 50 g/m² z.B. nach der Lehre der WO 03/018075, ein monolithisches Polyacrylat-System mit einem Wirkstoffgehalt von 5% zu beschichten, da das beschriebene System hinsichtlich der Dosiergenauigkeit voraussichtlich keine behördliche pharmazeutische Zulassung erhalten würde.

WO 0241878 beschreibt ein mehrlagiges transdermales therapeutisches System, aufgebaut aus einer Rückschicht, einer den Wirkstoff enthaltenden Schicht, einer Klebeschicht und einer Schutzschicht. Die Wirkstoffschicht kann aus einem Polymer, ausgewählt aus einer Vielzahl von verschiedenen Polymeren, unter anderem auch aus Polyisobutylen bestehen, die Klebeschicht aus Acryl-oder Methacrylharzen. Hauptaufgabe des beschriebenen Systems ist, eine genauere Wirkstoffdosierung zu erreichen; diese Aufgabe wird im Wesentlichen dadurch gelöst, dass mehrere kleine Pflastereinheiten zu einem größeren System zusammengefügt werden. Das System besitzt keine unterschiedlich dicken Wirkstoff- und Hautkontaktschichten bzw. solche mit unterschiedlichen Auftragsgewichten.

WO 02069940 beschreibt nikotinhaltige transdermale therapeutische Systeme, die sich durch besonders hohe Flexibilität auszeichnen. Sie bestehen aus einer Rückschicht, zwei nikotinhaltigen Matrixschichten und einer Schutzschicht, wobei die Matrixschichten nur aus (Meth-)Acrylat-Copolymeren unterschiedlicher Dicke bestehen. Es werden weder Angaben zu Auftragsgewichten noch zu deren Verhältnis zueinander gemacht.

Andererseits bevorzugt der Fachmann gut lösende und gut klebende Polymere für die Hauthaftung, um die Akzeptanz und Bioverfügbarkeit solcher Systeme zu verbessern. Als hauthaftende Polymere haben sich in den letzten Jahren Polyacrylate, welche als Nachteil allerdings hohes Lösungsvermögen für die üblicherweise für die transdermale Therapie in Frage kommenden pharmazeutischen Wirkstoffe aufweisen, als ideal herausgestellt.

Aufgabe der vorliegenden Erfindung ist daher die Bereitstellung eines transdermalen therapeutischen Systems mit einer Acrylatcopolymer-Hautkontaktschicht, einer gegenüber dem Stand der Technik verbesserten Wirkstoffausnutzungsrate und einer gegenüber dem Stand der Technik erhöhten Dosiergenauigkeit.

Die Aufgabe wird erfindungsgemäß gelöst durch Bereitstellung eines transdermalen therapeutischen Systems (TTS), umfassend eine für den Wirkstoff im wesentlichen undurchlässigen Rückschicht, eine hautferne Schicht auf der Basis von Polyisobutylenen, eine klebende Hautkontaktschicht auf der Basis von Acrylatcopolymeren, welche dünner ist als die hautferne Schicht und nach der Herstellung des TTS den überwiegenden Teil des Wirkstoffs enthält, sowie eine abziehbare, für den Wirkstoff im wesentlichen undurchlässigen Schutzschicht. Die hautferne Schicht weist erfindungsgemäß ein Auftragsgewicht von 100-200 g/m² auf; die Hautkontaktschicht weist erfindungsgemäß ein Auftragsgewicht von 20-30 g/m² auf.

In einer bevorzugten Ausführungsform beträgt das Verhältnis des Auftragsgewichtes der hautfernen Schicht zu der der Hautkontaktschicht mindestens 2:1, besonders bevorzugt 3:1 bis 5:1. Obwohl das als Basis der hautfernen Schicht verbundene Polyisobutylen eine niedrigere Diffusibilität als das gemäß dem Stand der Technik verwendete Polysiloxan aufweist, zeigt sich überraschenderweise, daß das erfindungsgemäße transdermale therapeutische System den aus dem Stand der Technik bekannten Systemen hinsichtlich Wirkstoffausnutzungsrate und Dosiergenauigkeit überlegen ist.

Das vorstehend beschriebene TTS kann folgendermaßen hergestellt werden:

Der Wirkstoff bzw. das Wirkstoffgemisch wird in einem geeigneten flüchtigen Lösungsmittel oder in einem Gemisch solcher Lösungsmittel gelöst, die erhaltene Lösung mit der für die hautferne Schicht bestimmten Polymermasse auf der Basis von Polyisobutylenen gemischt, die erhaltene Mischung in einer Schichtdicke von mindestens 200 µm (entsprechend 200 g/m²) auf eine geeignete Unterlage, beispielsweise eine silikonisierte Kunststoffschicht gleichmäßig aufgebracht. Nach Trocknen und Abdampfen der Lösungsmittelanteile wird die wirkstoffhaltige Polymerschicht mit einer weiteren Folie abgedeckt, welche die spätere Rückschicht des erfindungsgemäßen TTS darstellt. Auf das resultierende Laminat wird nach Entfernen der als Unterlage dienenden Kunststofffolie eine wirkstofffreie, auf AcrylatCopolymeren basierende klebende Hautkontaktschicht sowie eine abziehbare Schutzfolie in der Weise laminiert, daß die wirkstoffhaltige hautferne Schicht und die klebende Hautkontaktschicht aufeinanderliegen. Nach dem sofort einsetzenden Diffusionsausgleich migriert der größte Teil des bzw. der Wirkstoff(e) aus der hautfernen in die Hautkontaktschicht.

Die vorstehend beschriebene Erfindung ermöglicht eine sehr genaue und effiziente Dosierung des Wirkstoffes oder der Wirkstoffe durch das Einbringen des oder der letzteren in die Polymermasse aus Polyisobutylenen, welche einerseits in verhältnismäßig großer Schichtdicke aufgetragen wird und andererseits ein sehr geringes Lösungsvermögen für die erfindungsgemäß verwendeten Wirkstoffe aufweist, sowie sehr hohe Genauigkeit bei der Beschichtung einer Folie mit der Wirkstoff-/Polymermischung aufgrund der relativ hohen Schichtdicke. Es ergeben sich weitere Vorteile aus der Tatsache, daß die klebende Hautkontaktschicht auf der Basis von Acrylatcopolymeren nicht mit dem oder den Wirkstoff(en) beschichtet werden muß, so daß die Dicke dieser Schicht unter das nach dem bisherigen Stand der Technik übliche und für eine genaue Dosierung erforderliche Auftragsgewicht von über 50 g/m² reduziert und bevorzugt sogar auf ein typisches Auftragsgewicht von 20-30 g/m² beschränkt werden kann.

Als für den bzw. die Wirkstoff(e) im wesentlichen undurchlässige Rückschicht dient eine gegebenenfalls mit einem Polyisobutylen, einem Polyacrylat oder Polysiloxan beschichtete Polyethylenterephthalatfolie.

Die mit der Haut in Kontakt stehende Matrixschicht (Hautkontaktschicht) besteht aus Acrylatcopolymeren. Darunter sind zu verstehen Copolymere aus monomerer Acrylsäure, Methacrylsäure und/oder geeigneten Derivaten und gegebenenfalls monomeren Vinylverbindungen. Beispielhaft seien folgende Monomere genannt: Acrylsäure, Methacrylsäure, Acrylsäure- und Methacrylsäureester, z.B. insbesondere n-Butylacrylat, n-Butylmethacrylat, Ethylacrylat, 2-Ethylhexylacrylat, Ethylmethacrylat, Methylacrylat, Methylmethacrylat, tert.-Butylacrylat, sec.-Butylacrylat, tert.-Butylmethacrylat, Cyclohexylmethacrylat, 2-Ethylhexylmethacrylat, Isobornylmethacrylat, Isobutylmethacrylat, Isopropylacrylat, Isopropylmethacrylat und Mischungen dieser Monomere. Es handelt sich bei diesen Monomeren um Ester der Acryl- bzw. Methacrylsäure, die lineare, verzweigte oder cyclische aliphatische C1-C12-Substituenten besitzen. Diese Substituenten können ihrerseits z.B. durch Hydroxygruppen substituiert sein. Beispielhaft seien als Ester genannt 2-Hydroxyethylmethacrylat, 3-Hydroxypropylacrylat und 3-Hydroxypropylmethacrylat. Als geeignete Vinylverbindung sei Vinylacetat genannt.

Für die Zwecke der vorliegenden Erfindung sind vor allem solche Wirkstoffe geeignet, die hochwirksam sind. Darunter versteht der Fachmann im allgemeinen Wirkstoffe, deren Tagesdosis im mg-Bereich liegt, z.B. 1-500 mg. Erfindungsgemäß bevorzugt sind jedoch Wirkstoffe, deren Tagesdosis höchstens 30 mg beträgt.

Geeignete Wirkstoffe für die Verwendung im erfindungsgemäßen TTS sind beispielsweise Analgetika, Broncholytika, Antidiabetika, Vasodilatoren, Suchtentwöhnungsmittel und Parkinsonmittel.

Das erfindungsgemäße TTS ist jedoch insbesondere geeignet für die Schmerztherapie, bevorzugt mit Wirkstoffen aus der Gruppe der Opioide. Zu dieser Gruppe von pharmazeutischen Wirkstoffen zählen u.a. Morphin, Heroin und weitere Derivate des Morphins; Dihydromorphinderivate wie Hydromorphon (Dihydrocodein), Oxycodon; Morphinderviate wie Levorphanol, Buprenorphin; Analgetika der Pethidin-Gruppe, wie Pethidin, Ketobemidon, Loperamid, Diphenoxylat; Methadon und Derivate wie Levomethadon, Dextromoramit, Dextropropoxyphen; Fentanyl und seine Derivate (z.B. Alfentanil, Sufentanil, Remifentanil), Benzomorphanderivate wie Pentazocin und Phenylaminocyclohexinylderivate wie Tilidin; Tramadol. Für die Behandlung von Durchbruchschmerzen werden insbesondere schnell und kurz wirksame Opioide wie Morphin, Tramadol, Tilidin, Oxycodon, Hydromorphon, Buprenorphin, Fentanyl und Levomethadon bevorzugt.

Aus der Gruppe der Analgetika kommen ferner beispielsweise folgende in Betracht: Metamizol, Phenazon, Propyphenazon, Flupirtin, Nefopam, aus der Gruppe der AntiEpileptika Carbamazepin, Gabapentin, Clonazepam, ferner Antidepressiva wie Amitryptilin.

Die Erfindung schließt auch die Verwendung von Wirkstoffkombinationen, bestehend aus zwei oder mehreren Arzneistoffen, mit ein, insbesondere Kombinationen der vorstehend genannten Analgetika.

Die Polymerschichten, vorzugsweise die Hautkontaktschicht des erfindungsgemäßen TTS können ferner verschiedene Hilfs- oder Zusatzstoffe enthalten, beispielsweise aus der Gruppe der Lösungsvermittler, Lösungsmittel, Weichmacher, Klebrigmacher, Permeationsverbesserer, pH-Regulatoren, Antioxidanten und Konservierungsmittel.

Die Erfindung wird nachfolgend anhand von Beispielen erläutert:

### Beispiel 1:

Eine Lösung von Polyacrylsäure-co-(2-ethylhexyl)acrylat-co-vinylacetat, 30 % (g/g) in Ethylacetat wird in einer Schichtdicke von ca. 65-70 µm auf eine silikonisierte Polyethylenterephthalat-Folie (100 µm Dicke) beschichtet und 2 Stunden offen bei 25°C getrocknet, so dass eine Kleberschicht von 20 g/m² resultiert. In einem separaten Ansatz werden 2g Fentanyl-Base, 70 g Polyisobutylen-100, 28 g Polyisobutylen-10, 100 g Methyl-Ethylketon und 200 g n-Heptan in eine viskose Lösung gebracht und noch 2 Stunden gerührt. Diese Phase wird mit einer Schichtdicke von etwa 500 µm auf eine 15µm dicke Folie aus Polyethylenterephthalat beschichtet, so dass eine wirkstoffhaltige trockene Kleberschicht von 100 g/m² ensteht. Diese Schicht wird kleberseitig mit der zuvor gefertigten Acrylatcopolymer-Schicht (Klebschicht auf Klebschicht) so laminiert, dass eine zusammenhängende, zweischichtige Matrix entsteht. Nach Ablösen der Schutzfolie entsteht ein klebefähiges System, welches mit der Polyacrylatseite auf die Haut aufgeklebt werden kann. Das System kann durch geeignete Stanzeinrichtungen auf die erforderliche Größe gestanzt werden.

### Beispiel 2:

Eine Lösung von Poly(2-ethylhexyl)acrylat-co-vinylacetat-co-(2-hydroxyethyl)acrylat-co-(2, 3-epoxpropyl)methacrylat, 30 % (g/g) in Ethylacetat wird in einer Schichtdicke von ca. 65-70 µm auf eine silikonisierte Polyethylenterephthalat-Folie (100 µm Dicke) beschichtet und 2 Stunden offen bei 25°C getrocknet, so dass eine Kleberschicht von 20 g/m² resultiert. In einem separaten Ansatz werden 1,5 g Fentanyl-Base, 70 g Polyisobutylen-100, 28 g Polyisobutylen-10, 100 g Methyl-Ethylketon und 200 g n-Heptan in eine viskose Lösung gebracht und noch 2 Stunden gerührt. Diese Phase wird mit einer Schichtdicke von etwa 500 µm auf eine 15µm dicke Folie aus Polyethylenterephthalat beschichtet, so dass eine wirkstoffhaltige trockene Kleberschicht von 100 g/m² entsteht.

Diese Schicht wird kleberseitig mit der zuvor gefertigten Acrylatcopolymer-Schicht (Klebschicht auf Klebschicht) so laminiert, dass eine zusammenhängende, zweischichtige Matrix entsteht. Nach Ablösen der Schutzfolie entsteht ein klebefähiges System, welches mit der Polyacrylatseite auf die Haut aufgeklebt werden kann. Das System kann durch geeignete Stanzeinrichtungen auf die erforderliche Größe gestanzt werden.

## Patentansprüche

1. Transdermales therapeutisches System zur Verabreichung von mindestens einem bei Raumtemperatur nicht flüchtigen pharmazeutischen Wirkstoff in einer Tagesdosis von höchstens 30 mg, umfassend eine hautabgewandte, für den oder die Wirkstoff(e) undurchlässige Rückschicht, eine angrenzende hautferne Schicht, eine an die hautferne Schicht angrenzende klebende Hautkontaktschicht und eine von der letzteren abziehbare, für den oder die Wirkstoff(e) undurchlässige Schutzschicht, **dadurch gekennzeichnet, dass**
a) die Hautkontaktschicht aus Acrylatcopolymeren besteht, ein Auftragsgewicht von 20 - 30 g/m² aufweist und den überwiegenden Teil des oder der bei der Herstellung des Systems in die hautferne Schicht eingebrachten Wirkstoffs oder Wirkstoffe enthält und
b) die hautferne Schicht aus Polyisobutylenen besteht und ein Auftragsgewicht von 100 - 200 g/m² aufweist.

2. Transdermales therapeutisches System gemäß Anspruch 1, **dadurch gekennzeichnet, dass** das Verhältnis des Auftragsgewichtes der hautfernen Schicht zu dem der Hautkontaktschicht mindestens 2:1 beträgt.

3. Transdermales therapeutisches System gemäß Anspruch 2, **dadurch gekennzeichnet, dass** das Verhältnis 3:1 bis 5:1 beträgt.

4. Transdermales therapeutisches System gemäß mindestens einem der vorhergehenden Ansprüchen, **dadurch gekennzeichnet, dass** der oder die Wirkstoff(e) nach Herstellung des Systems und Diffusionsausgleich zum überwiegenden Teil aus der hautfernen Schicht in die Hautkontaktschicht migrieren.

5. Transdermales therapeutisches System gemäß Anspruch 1, **dadurch gekennzeichnet, dass** die Polymerbasis der Hautkontaktschicht aus Copolymeren der Monomeren ausgewählt aus der Gruppe Acrylsäure, Methacrylsäure, Acrylsäureester, Methacrylsäureester und Vinylverbindungen besteht.

6. Transdermales therapeutisches System gemäß Anspruch 5, **dadurch gekennzeichnet, dass** die Vinylverbindung Vinylacetat ist.

7. Transdermales therapeutisches System gemäß Anspruch 1, **dadurch gekennzeichnet, dass** der oder die Wirkstoffe Analgetika sind.

8. Transdermales therapeutisches System gemäß Anspruch 7, **dadurch gekennzeichnet, dass** mindestens einer der Wirkstoffe ein Opioid ist.

9. Transdermales therapeutisches System gemäß Anspruch 8, **dadurch gekennzeichnet, dass** das Opioid Fentanyl und/oder eines seiner Derivate ist.

10. Transdermales therapeutisches System gemäß Anspruch 9, **dadurch gekennzeichnet, dass** das Fentanylderivat Alfentanil, Sufentanil oder Remifentanil ist.

11. Verfahren zur Herstellung eines transdermalen therapeutischen Systems gemäß Anspruch 1 **dadurch gekennzeichnet, dass** der oder die Wirkstoff(e) gelöst in einem geeigneten flüchtigen Lösungsmittel oder Lösungsmittelgemisch mit der für die hautferne Schicht bestimmten Polymermasse auf der Basis von Polyisobutylenen gemischt wird bzw. werden, die erhaltene Mischung in einer Schichtdicke von mindestens 200 µm auf eine silikonisierte Kunststofffolie gleichmäßig aufgebracht wird, das erhaltene Laminat nach Trocknen und Abdampfen des Lösungsmittels mit der Rückschicht abgedeckt wird und nach Entfernen der silikonisierten Kunststoff-Folie auf die hautferne Schicht die klebende Hautkontaktschicht sowie die abziehbare Schutzschicht in der Weise auflaminiert werden, dass die wirkstoffhaltige hautferne Schicht und die klebende Hautkontaktschicht aufeinanderliegen.

12. Verfahren gemäß Anspruch 11, **dadurch gekennzeichnet, dass** hautferne Schicht und Hautkontaktschicht derart aufeinanderlaminiert werden, daß die Migration des Wirkstoffs oder der Wirkstoffe aus der ersteren in die letztere ermöglicht wird.

13. Verfahren gemäß den Ansprüchen 11 bis 12 **dadurch gekennzeichnet, dass** die hautferne Schicht ein Auftragsgewicht von 100 - 200 g/m² und die Hautkontaktschicht ein Auftragsgewicht von 20 - 30 g/m² aufweist.

14. Verfahren gemäß Anspruch 13, **dadurch gekennzeichnet, dass** das Verhältnis des Auftragsgewichtes der hautfernen Schicht zu dem der Hautkontaktschicht mindestens 2:1 beträgt.

15. Verfahren gemäß Anspruch 14, **dadurch gekennzeichnet, dass** das Verhältnis 3:1 bis 5:1 beträgt.

16. Verfahren gemäß den Ansprüchen 11-15, **dadurch gekennzeichnet, dass** der Wirkstoff Fentanyl oder ein Fentanylderivat ist.

17. Verfahren gemäß Anspruch 16, **dadurch gekennzeichnet, dass** das Fentanylderivat Alfentanil, Sufentanil oder Remifentanil ist.

## Claims

1. A transdermal therapeutic system for administration of at least one non-volatile at room temperature pharmaceutical active ingredient at a daily dose of at most 30 mg which comprises a skin facing away impermeable for the or each active ingredient(s) backing layer, and adjoining remote from the skin layer, one applied to the skin-remote layer adhesive skin contact layer and a peelable from the latter, impermeable to the or each active ingredient(s) protective layer, **characterized in that**
a) the skin contact layer is based on acrylic copolymers, has a coating weight of 20-30 g/m² and contains the predominant part of the or each active ingredient(s) incorporated into the remote from the skin layer in the production of the system and
b) the remote from the skin layer is based on polyisobutylenes and has a coating weight of 100-200 g/m².

2. The transdermal therapeutic system according to claim 1, **characterized in that** the ratio of the coating weight of the remote from the skin layer to the skin contact layer is at least 2:1.

3. The transdermal therapeutic system according to claim 2, **characterized in that** the ratio is 3:1 to 5:1.

4. The transdermal therapeutic system according to at least one of the preceding claims, **characterized in that** the or each active ingredient(s) after the production of the system and compensating diffusion migrate for the predominant part from the remote from the skin layer into the skin contact layer.

5. The transdermal therapeutic system according to claim 1, **characterized in that** the polymer base of the skin contact layer is selected from copolymers of monomers selected from the group of acrylic acid, methacrylic acid, acrylic esters, methacrylic esters and vinyl compounds.

6. The transdermal therapeutic system according to claim 5, **characterized in that** the vinyl compound is vinyl acetate.

7. The transdermal therapeutic system according to claim 1, **characterized in that** the or each active ingredient(s) are analgesics.

8. The transdermal therapeutic system according to claim 7, **characterized in that** at least one of the active ingredients is an opioid.

9. The transdermal therapeutic system according to claim 8, **characterized in that** the opioid is fentanyl and/or one of its derivatives.

10. The transdermal therapeutic system according to claim 9, **characterized in that** the fentanyl derivate is alfentanil, sufentanil, or remifentanil.

11. A process for producing a transdermal therapeutic system according to claim 1, **characterized in that** the one or more active ingredient(s) dissolved in a suitable volatile solvent or solvent mixture is mixed with the particular for remote from the skin layer polymer composition on the basis of polyisobutylenes or are the obtained mixture in a layer thickness of at least 200 microns is uniformly applied to a siliconized plastic film, the resulting laminate, after drying and evaporation of the solvent, is covered with the backing layer, and after removal of the siliconized plastic film to the remote from the skin layer, the adhesive skin contact layer and the peelable protective layer are laminated in such a way that the active ingredient-containing remote from the skin layer and the adhesive skin contact layer lie on one another.

12. The process according to claim 11, **characterized in that** remote from the skin layer and the skin contact layer are laminated in a way that the migration of the or each active ingredient(s) from the former is made possible into the latter.

13. The process according to claims 11 and 12 **characterized in that** the layer remote from the skin has a coating weight of 100-200 g/m² and the skin contact layer has a coating weight of 20-30 g/m².

14. The process according to claim 13, **characterized in that** the ratio of the coating weight of the remote from the skin layer to the contact layer is at least 2:1.

15. The process according to claim 14, **characterized in that** the ratio is 3:1 to 5:1.

16. The process according to claims 11-15, **characterized in that** the active ingredient is fentanyl or a fentanyl derivate.

17. The process according to claim 16, **characterized in that** the fentanyl derivate is alfentanil, sufentanil, or remifentanil.

## Revendications

1. Système thérapeutique transdermique destiné à l'administration d'au moins une substance active pharmaceutique non volatile à température ambiante à une dose quotidienne maximale de 30 mg, comprenant une couche arrière imperméable à la ou les substance(s) active(s), opposée à la peau, une couche adjacente éloignée de la peau, une couche adhésive en contact avec la peau et adjacente à la couche éloignée de la peau et une couche de protection imperméable à la ou les substance(s) active(s), enlevable de la dite couche adhésive, **caractérisé en ce que**
a) la couche en contact avec la eau est composée de copolymères acryliques, présente un poids d'application de 20 à 30 g/m² et contient la partie principale de la ou des substance(s) active(s) apportée(s) dans la couche éloignée de la peau lors de la fabrication du système et
b) la couche éloignée de la peau est composée de polyisobutylènes et présente un poids d'application de 100 à 200 g/m².

2. Système thérapeutique transdermique selon la revendication 1, **caractérisé en ce que** le rapport du poids d'application de la couche éloignée de la peau à celui de la couche en contact avec la peau est au moins de 2:1.

3. Système thérapeutique transdermique selon la revendication 2, **caractérisé en ce que** le rapport est de 3:1 à 5:1.

4. Système thérapeutique transdermique selon au moins une des revendications précédentes, **caractérisé en ce que** la ou les substance(s) active(s) migrent après la fabrication du système et la compensation de diffusion jusqu'à la partie principale de la couche éloignée de la peau dans la couche en contact avec la peau.

5. Système thérapeutique transdermique selon la revendication 1, **caractérisé en ce que** la base polymère de la couche en contact avec la peau est composée de copolymères des monomères choisis dans un groupe d'acide acrylique, d'acide méthacrylique, d'ester d'acide acrylique, d'ester d'acide méthacrylique et de composés vinyliques.

6. Système thérapeutique transdermique selon la revendication 5, **caractérisé en ce que** le composé vinylique est de l'acétate de vinyle.

7. Système thérapeutique transdermique selon la revendication 1, **caractérisé en ce que** la ou les substance(s) active(s) sont des analgésiques.

8. Système thérapeutique transdermique selon la revendication 7, **caractérisé en ce qu'**au moins une des substances actives est un opioïde.

9. Système thérapeutique transdermique selon la revendication 8, **caractérisé en ce que** l'opioïde est le fentanyl et/ou un de ses dérivés.

10. Système thérapeutique transdermique selon la revendication 9, **caractérisé en ce que** le dérivé de fentanyl est de l'alfentanil, du sufentanil ou du rémifentanil.

11. Procédé de fabrication d'un système thérapeutique transdermique selon la revendication 1, **caractérisé en ce que** la ou les substance(s) active(s) dissoute(s) dans un solvant volatil approprié ou un mélange de solvants est ou sont mélangée(s) à la masse polymère à base de polyisobutylènes qui est définie pour la couche éloignée de la peau, le mélange obtenu est appliqué de manière homogène sur une feuille en matière plastique siliconée dans une épaisseur de couche d'au moins 200 µm, le laminé obtenu est recouvert, après séchage et évaporation du solvant, avec la couche arrière et, la couche adhésive en contact avec la peau et la couche de protection enlevable sont laminées après avoir enlevé la feuille de matière plastique siliconée sur la couche éloignée de la peau de manière à ce que la couche éloignée de la peau qui contient la substance active et la couche adhésive en contact avec la peau soient disposées l'une sur l'autre.

12. Procédé selon la revendication 11, **caractérisé en ce que** la couche éloignée de la peau et la couche en contact avec la peau sont laminées l'une sur l'autre de telle sorte que la migration de la ou des substance(s) active(s) soit possible de la première vers la dernière.

13. Procédé selon les revendications 11 à 12, **caractérisé en ce que** la couche éloignée de la peau présente un poids d'application de 100 à 200 g/m² et la couche en contact avec la peau un poids d'application de 20 à 30 g/m².

14. Procédé selon la revendication 13, **caractérisé en ce que** le rapport du poids d'application de la couche éloignée de la peau à celui de la couche en contact avec la peau est au moins de 2:1.

15. Procédé selon la revendication 14, **caractérisé en ce que** le rapport est de 3:1 à 5:1.

16. Procédé selon les revendications 11 à 15, **caractérisé en ce que** la substance active est le fentanyl ou un dérivé de fentanyl.

17. Procédé selon la revendication 16, **caractérisé en ce que** le dérivé de fentanyl est de l'alfentanil, du sufentanil ou du rémifentanil.
